# EUROPEAN PATENT APPLICATION

(11) **EP 1 618 867 A1**
(43) Date of publication of application: **25.01.2006**
(21) Application number: 04254328.0
(22) Date of filing: 20.07.2004
(51) Int. Cl.: A61K 8/34, A61K 8/42, A61K 8/44, A61K 8/49, A61K 8/67, A61Q 19/00

(54) **Topical compositions comprising a vitamin B3 compound and their use for treating dry skin**

(71) Applicant: THE PROCTER & GAMBLE COMPANY, Cincinnati, Ohio 45202 (US)
(72) Inventor: Matts, Paul Jonathan, Addlestone Surrey KT15 2HT (GB)
(74) Representative: L'Huillier, Florent Charles

(57) **Abstract**

The present invention relates to a method of breaking the cycle of dry skin with a composition comprising a moisturizer and a Vitamin B3 compound.

## Description

### FIELD

The present invention relates to skin moisturizing products and methods (cosmetic and therapeutic) of treating dry skin.

### BACKGROUND

Many cosmetic compositions have been proposed to treat dry skin. These compositions typically contain lipophilic moisturizing agents that inhibit water loss via occlusion or hydrophilic moisturizing agents that trap water inside the skin.

However these compositions often only offer a temporary relief for dry skin sufferers. It would be therefore desirable to provide a method for improving skin conditions on a longer term basis.

It has long been the general assumption that dry skin condition was a static condition that could only be temporary relieved by the application of moisturizing / skin repairing composition. The inventor has now found that dry skin conditions can be more accurately described as a self-perpetuating cycle, and that use of a composition comprising a moisturizer and vitamin B3 compound can break this cycle.

### SUMMARY

The present invention is directed to the use of a composition comprising a moisturizer, preferably glycerine, and a vitamin B3 compound to break the cycle of dry skin.

The Inventor has discovered that the cycle of dry skin comprises one Induction Step (I) and four self-perpetuating cycle steps (A, B, C, D). The Induction Step is the initial degradation of the condition from healthy skin. Various factors can initiate this degradation.

Step A can be described as the initiation and perpetuation of a mini-cycle of barrier deterioration. Step B can be described as the induction of a hyper-proliferative state. Step C is the development of abnormal, dysfunctional stratum corneum components. Step D is the loss in efficiency of desquamation and ensuing scaling, thickening and loss of hygroscopicity of the stratum corneum.

The present invention also relates to a packaging containing a composition, said composition comprising a moisturizer, preferably glycerine, and a vitamin B3 compound, preferably Niacinamide, wherein the packaging comprises an indication for the user that the composition in the packaging breaks the cycle of dry skin. The indication may be a message on a label on the container, e.g. "helps breaking the cycle of dry skin", or "proven to break the cycle of dry skin".

The present invention also relates to the use of a composition comprising a moisturizer, preferably glycerine, and a vitamin B3 compound, preferably Niacinamide, for the manufacture of a therapeutic composition for breaking the cycle of dry skin.

### BRIEF DESCRIPTION OF THE FIGURE

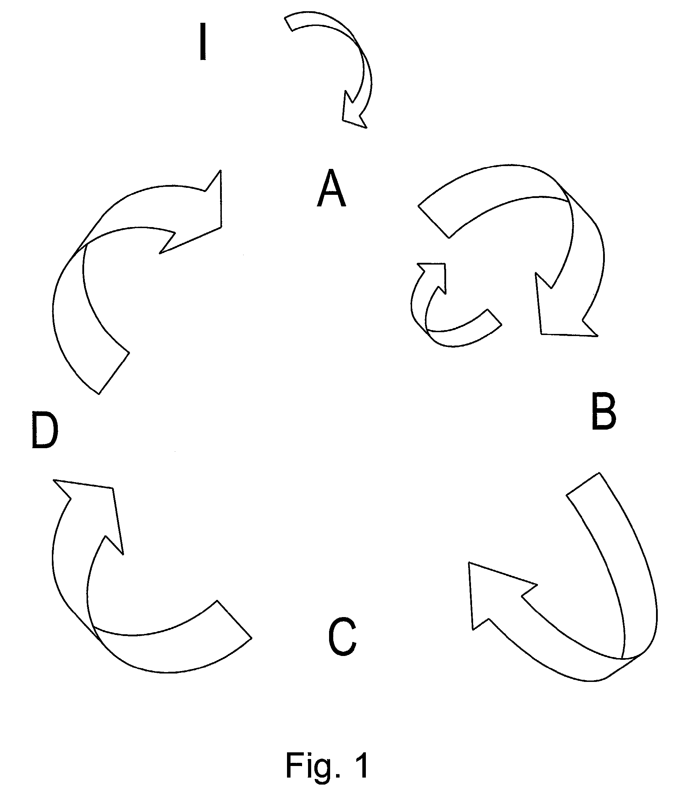

Fig. 1 is a schematic diagram of the cycle of dry skin, showing the Induction Step (I) and the four recurring cycle steps (A, B, C, D);

### DETAILED DESCRIPTION

All weights, measurements and concentrations herein are measured at 25°C on the composition in its entirety, unless otherwise specified.

Unless otherwise indicated, all percentages of compositions referred to herein are weight percentages and all ratios are weight ratios.

Except where specific examples of actual measured values are presented, numerical values referred to herein should be considered to be qualified by the word "about".

Unless otherwise indicated, the content of all literature sources referred to within this text are incorporated herein in full by reference.

As used herein, the acronym "SC" refers to the stratum corneum, which describes the outer layer of human epidermis, being composed of several layers of tightly-bound corneocytes and associated intercellular material.

As used herein, the acronym "NMF" refers to Natural Moisturizing Factors, which are a mixture of hygroscopic amino acids, salts and sugars that help maintain stratum corneum hydration and, therefore, plasticity.

### Induction

The induction and propagation of "dry skin" may be expressed intuitively as a cyclical technical model, dependent on SC integrity and particularly upon barrier function and homeostasis, essential for controlling SC water flux and content (see Fig.1 for a schematic diagram of the model).

The induction phase can be mediated by a variety of different factors:
- in general, ageing results in a reduction in SC ceramide levels and progressive impairment of barrier function and that is concurrent with a progressive increase in dry skin expression;
- surfactant dissolution of SC lipid, such as may occur as a consequence of everyday cleansing or occupational exposure, has long been known to be a major cause of barrier disruption and ensuing dry skin symptoms. Soap-induced dry skin is associated with reduced ceramide levels, together with increased fatty acid levels probably from deposited fatty acid soaps. These lipid compositional changes result in gross abnormalities in SC lipid arrangement and probably contribute to a greater leaching of "Natural Moisturising Factors" (NMF) from the SC. Even exposure to water alone can damage the barrier and leach out NMF from the SC. In this way, SC barrier lipids help prevent loss of NMF and maintain optimal water activity in the SC, allowing a variety of enzymes to process structural components in this tissue, thus enabling it to cope with a desiccating environment and yet still be able to desquamate.
- low environmental temperature and humidity (consistent with seasonal variation) also have a strong association with barrier dysfunction and dryness. Reduced levels of SC lipids have been reported in the winter months of the year compared with the summer months.
- modern indoor climate-controlled environments are also becoming recognised as drivers of dry skin conditions, probably due to the low relative humidity of the air recirculated, particularly in subjects whose skin has been conditioned to a high humidity climate, due to the reduced ceramide levels, NMF levels and desquamatory enzyme levels.
- psychological stress has recently been shown to be a mediator of barrier homeostasis and ensuing dry skin consequences. These types of stressors result in reduced SC ceramide levels, reduced NMF levels and smaller corneocytes.
- genetics can also be involved in the expression of dry skin conditions. At worst, atopics are known to have reduced ceramide and NMF levels due to differences in ceramide processing enzymes and reduced expression of filaggrin.
- pH will also influence SC barrier function as higher SC pH reduces the activity of ceramide processing enzymes.
   Once the skin has been provoked by one or more of the mechanisms above, the inventor has found that an inevitable sequence of events is triggered: the cycle of dry skin.

### Step A: A mini-cycle of barrier deterioration is initiated and perpetuated:

Normal, healthy SC contains approximately 10-20% water. This is dependent upon:
(a) the quantity and flux of water passing from the lower layers of the SC, itself dependent upon diffusion from the underlying viable epidermis. The water concentration in the lowest SC layer has been estimated at 49M, equivalent to that of the viable epidermis;
(b) the flux of water that is eliminated by evaporative water loss across the SC barrier which is highly dependent on SC lipids and the tortuosity of the SC architecture;
(c) the hygroscopicity of the SC, dependent upon (a) and (b) and also the "Natural Moisturising Factor" complement within the SC.

Trans-Epidermal Water Loss (TEWL), therefore, for a given body site, corresponds to the passive diffusion of water vapour through the epidermis and SC and depends on the gradient of water concentration across these structures. As the water concentration in the upper layers of normal SC has been estimated at approximately 12M (at 40% humidity and 31°C), this gradient spans approximately 37M.

It has been estimated that the SC loses its flexibility once its water content falls below approximately 10%, which may be caused by one or a combination of the factors noted in the introduction above. Without intervention, this quickly leads to a steeper SC hydration gradient, a decrease in net recondensation on the SC surface, a corresponding increase in evaporative water loss from the SC surface, a consequent further drop in SC water concentration and so on. The inevitable rapid consequence of this series of events is a decrease in the plastic or viscous properties of the SC (commonly interpreted as skin "softness" or "suppleness"), an increase in SC fragility /brittleness and an impairment of SC barrier function. This surface dehydration is the first step in the development of the dry skin cycle and is further exacerbated by destruction of the normal barrier lipid lamellae in the outer layers of the SC during bathing. The impaired barrier in the superficial layers of the SC allows leaching of NMF from the outermost skin cells, thereby reducing SC water activity. Whiteness between the dermatoglyphics (caused by backscatter from multiple tissue-air interfaces) and minor scaling due to the dehydration of individual corneocytes are the first visible steps in the cycle. Perturbation to the barrier then leads to further development of dry skin.

Due to the cyclical nature of these processes, therefore, it becomes virtually impossible to distinguish between dry skin conditions that are provoked initially by barrier disruption or by dehydration of the SC.

Once the barrier has been disrupted, even superficially, a new cascade of events is started:

### Step B: Induction of a hyper-proliferative state

Acute and chronic insults to the SC barrier will lead to enhanced keratinocyte proliferation, consequent hyperkeratosis and mild inflammatory changes, one of the hall-marks of dry skin conditions, as the skin attempts to repair itself. This response is mediated via production and secretion of cytokines and growth factors, many researchers citing the ratio between interleukin one-alpha (IL-1α) and interleukin one receptor antagonist as a key marker of this process. The degree of hyperprofileration has been shown to be dependent upon the corresponding degree of barrier perturbation, probably reflecting both the ingress of exogenous irritants through the impaired barrier and the growing realisation that the SC barrier is itself a biosensor, corneocytes and keratinocytes themselves participating in the release of these messengers. The hyperproliferation of the epidermis probably occurs as a result of the double paracrine (which refers to hormones that stimulate cells to release other locally-acting hormones - see example in next sentence) signaling events between the epidermis and dermis. IL-1 acts on fibroblasts which in turn secrete KGF and GMCSF inducing hyperproliferation and dysfunctional differentiation of keratinocytes.

The induction of this inflammatory hyperproliferative state is absolutely key in the cycle of dry skin as it fundamentally leads to aberrant differentiation and the over-hasty production of a variety of poor quality materials and structures vital to the proper functioning of the SC barrier and normal healthy skin.

### Step C: Development of abnormal, dysfunctional SC components

(a) Immature corneocyte envelopes
   The SC itself undergoes a natural maturation process, but this is disturbed in hyperproliferative conditions. During differentiation, corneocytes are first formed as a fragile form and are progressively rigidified by the action of transglutaminase, further cross linking the corneocyte envelope and attaching covalently bound lipids (ceramides, fatty acids and cholesterol) to free carboxyl groups on involucrin (the major corneocyte envelope protein). In normal skin, therefore, corneocytes reaching the surface of the SC have predominantly rigid envelopes. However, in hyperproliferative states such as chronic dry skin, there is a significant increase in the ratio of fragile envelopes. These immature corneocytes also are less hydrophobic. This is highly significant, as fully mature hydrophobic envelopes are essential for barrier lipid binding; immature, more hydrophilic envelopes tend to produce a weaker barrier.
(b) Abnormal ceramides
   Barrier lipids are also affected in chronic hyperproliferative states. In response to barrier perturbation, ceramide synthesis is upregulated as a direct repair mechanism. It is now known, however, that in dry skin, there is an accumulation of sphingosine- containing ceramides at the expense of phyto-sphingosine ceramides, apparently contributing to, rather than ameliorating, the problem.
(c) Reduction in expression / activity of desquamatory enzymes in the stratum corneum
   It is known that stratum corneum chymotryptic enzyme (SCCE) specific activity within the SC is reduced in superficial dry skin conditions due to either changes in SC lipid architecture, reduced SC water content or by denaturation of the enzyme itself. Reduced water activity of the SC can further reduce SCCE activity and desquamation which thereby contributes significantly to all stages of the dry skin cycle. In chronic dry skin conditions increased proteolytic activities have been reported but as the SC still shows scaling these enzymes are still not allowing desquamation to proceed efficiently. Expression of these enzymes in the epidermis is also associated with inflammation and itching. Other enzymes are reported to be involved in desquamation but as yet their role in expression of dry skin has not been established fully.
(d) Immature smaller corneocytes
   Finally, the physical barrier of the SC is dependent largely upon the enforced highly convoluted, tortuous path around corneocytes, providing an approximate 10-fold increase in SC transit time. In hyperproliferative states, the projected size of the corneocytes decreases, reducing the tortuous pathway and, thus, compromising further the SC barrier.

### Step D: Loss in efficiency of desquamation and ensuing scaling, thickening and loss of hygroscopicity of the SC

SCCE plays a key role in the correct dissociation of corneocytes from one another in the process of SC desquamation, via proteolysis of peripheral corneodesmosomal linkages. Impaired activity of SCCE, and probably other proteases, therefore, automatically leads to a dramatic increase in scaling and a compaction of multiple layers of sheets of un-separated corneocytes. This is, of course, one of the obvious consumer-noticeable expressions of "dry skin".

The formation of a thicker SC with impaired desquamation has, again, immense biophysical importance. The water gradient across the thicker SC becomes steeper, leading to further increases in evaporative water loss, reducing further water concentration in the outer SC and propagating directly another round of the dry skin cycle.

Corneocytes that should have already been shed via desquamation, therefore, remain adhered to the SC surface for significantly longer periods of time. The resulting barrier protecting these corneocytes and their contents is now weaker due to changes in barrier lipid profiles and surface hydrophobicity. It is now known that the hygroscopic (though highly water-labile) NMF present within corneocytes of normal SC, are depleted gradually through normal everyday activities such as cleansing and / or occupational duties. The corneocytes of dry SC are, therefore, subject to exaggerated insult such as that just described, as a direct result of their increased residence time at the surface. The dry skin cycle, thus, is propagated further by an increased loss of NMF relative to normal skin and a corresponding loss in SC hygroscopicity.

Finally and most importantly, the development of an increasingly thick, dry SC results in a layer characterised, from a biomechanical viewpoint, by a dramatic increase in hardness and brittleness. The consumer perceives this as tightness. These properties create a SC barrier inherently susceptible to mechanical stress and fracture, another factor driving the impairment in barrier function cyclical nature of the dry skin cycle.

### Conclusion

The clinical endpoint of "dry skin" cannot be regarded as static but rather is most fully described as a cycle that, without intervention, tends to perpetuate itself. Pivotal to every stage of this cycle and its propagation is a compromised SC barrier. Interventions that truly break the dry skin cycle, therefore, by definition need not only to treat symptomatic manifestations, but repair and augment SC barrier function.

The inventor has found that the combination of a moisturizer and vitamin B3 compound can break the cycle of dry skin.

### Vitamin B3 and its compounds

As used herein, "vitamin B₃ compound" includes compounds having the formula: 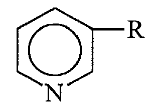
wherein R is - CONH₂ (i.e. niacinamide), - COOH (i.e. nicotinic acid) or - CH₂OH (i.e. nicotinyl alcohol); derivatives thereof; and salts of any of the foregoing. Exemplary derivatives of the foregoing vitamin B₃ compounds include nicotinic acid esters, including non-vasodilating esters of nicotinic acid, nicotinyl amino acids, nicotinyl alcohol esters of carboxylic acids, nicotinic acid N-oxide and niacinamide N-oxide.

Suitable esters of nicotinic acid include nicotinic acid esters of C₁-C₂₂, preferably C₁-C₁₆, more preferably C₁-C₆ alcohols. The alcohols are suitably straight-chain or branched chain, cyclic or acyclic, saturated or unsaturated (including aromatic), and substituted or unsubstituted. The esters are preferably non-vasodilating. As used herein, "non-vasodilating" means that the ester does not commonly yield a visible flushing response after application to the skin in the subject compositions (the majority of the general population would not experience a visible flushing response, although such compounds may cause vasodilation not visible to the naked eye). Non-vasodilating esters of nicotinic acid include tocopherol nicotinate and inositol hexanicotinate; tocopherol nicotinate is preferred.

Preferably, the Vitamin B3 compound is present in amount of from about 0.25% to about 6% by weight of the composition, more preferably of from about 0.5% to about 5%, even more preferably from about 1% to about 4%.

The inventor has found that the combination of a Vitamin B3 compound and a moisturizer synergistically breaks the cycle of dry skin.

### Moisturizers

Moisturizers useful herein are functionally described as compounds capable of increasing the water content of SC. This term includes hygroscopic moisturizers (also called humectants) that bind to water in the SC and occlusive moisturizers that trap water under the skin by providing a physical layer above the skin.

Hygroscopic moisturizers (humectants): suitable humectants useful herein include polyhydric alcohols, sodium 2-pyrrolidone-5-carboxylate (NaPCA), amino acids and derivatives, guanidine; glycolic acid and glycolate salts (e.g. ammonium and quaternary alkyl ammonium); lactic acid and lactate salts (e.g. ammonium and quaternary alkyl ammonium); other alpha hydroxy acids such as malic acid, aloe vera in any of its variety of forms (e.g. aloe vera gel); hyaluronic acid, precursors and derivatives thereof (e.g. glucosamine and salt derivatives such as sodium hyaluronate); lactamide monoethanolamine; acetamide monoethanolamine; urea; and mixtures thereof.

Preferred for use in the compositions of the present invention are polyhydric alcohols. Suitable polyhydric alcohols for use herein include poly-alkylene glycols and more preferably alkylene polyols and their derivatives, including propylene glycol, dipropylene glycol, polypropylene glycol, polyethylene glycol and derivatives thereof, sorbitol, hydroxypropyl sorbitol, erythritol, threitol, pentaerythritol, xylitol, glucitol, mannitol, hexylene glycol, butylene glycol (e.g., 1,3-butylene glycol), hexane triol (e.g., 1,2,6-hexanetriol), trimethylol propane, neopentyl glycol, glycerine, ethoxylated glycerine and propoxylated glycerine. Preferred polyhydric alcohols of the present invention are polyhydric alcohols with 3 to 9 carbon atoms in the molecule. Examples include glycerine, butylene glycol, propylene glycol, dipropylene glycol, polyethylene glycol and derivatives thereof, hexane triol, ethoxylated glycerine and propoxylated glycerine, and mixtures thereof. More preferred for use in the present invention is glycerine. The compositions of the present invention comprise from about 5% to about 40% polyhydric alcohol, preferably from about 7% to about 20%, more preferably from about 8% to about 15% by weight of the composition.

Occlusive moisturizers: A non-limiting list of occlusive moisturizers can be found in the International Cosmetic Ingredients Dictionary and Handbook, see for example Vol. 2 of the Eight Edition pages 1782-1785 ("Skin-conditioning agents - occlusive"). Preferred occlusive moisturizers for use herein are petrolatum, isohexadecane, isononyl isononanoate, methyl isostearate, isopropyl isostearate, and mixtures thereof.

When present, the compositions may comprise the occlusive moisturizers at a level of from about 0.1 % to about 10%.

### Other ingredients

The compositions of the present invention may be formulated as any skin care compositions and may comprise a wide variety of optional components, provided that such optional components are physically and chemically compatible with the essential components described herein, and do not unduly impair stability, efficacy or other use benefits associated with the compositions of the present invention. Typical cosmetic components that may be used are for example referenced in the International Cosmetic Ingredients Dictionary and Handbook, see for example its 8th. Edition , dated 2000.

The moisturizer and Vitamin B3 compound are preferably incorporated into an oil-in-water emulsion chassis, structured by a fatty alcohol lamellar gel network and thickened further using a polyacrylate polymeric thickener, producing an emulsion with excellent stability.

Dex-panthenol was found to be particularly useful as a co-active to the Vitamin B3 compound. Dex-panthenol has been used in a clinical setting for many years, in the treatment of various dermatoses, including burns, ulcers, eczema, surgical wounds and contact dermatitis. D-panthenol may be included in the compositions to augment the barrier-enhancing properties of the Vitamin B3 compound (e.g. Niacinamide).

As discussed previously, Natural Moisturizing Factors (NMF) play a critical role in maintaining corneocyte hygroscopicity. There is some evidence that topical supplementation with an artificial blend of NMF can help augment SC hygroscopicity . A commercial blend of NMF (Prodew 400®; Ajinomoto) may be incorporated into the compositions to help further augment SC hygroscopicity. The compositions preferably comprise a least one and preferably all NMFs present in Prodew 400®: glycine, alanine, proline, serine, threonine, arginine, lysine, glutamic acid, and sodium-2-pyrrolidone-5-carboxylate.

The topical compositions of the present invention may comprise a wide variety of further optional components, provided that such optional components are physically and chemically compatible with the essential components described herein, and do not unduly impair stability, efficacy or other use benefits associated with the compositions of the present invention.

### Examples

Exemplary compositions capable of breaking the cycle of dry skin are disclosed below:

| **INGREDIENTS** | **EXAMPLE 1** | **EXAMPLE 2** | **EXAMPLE 3** |
|---|---|---|---|
| | **%w/w** | **%w/w** | **%w/w** |
| DEIONISED WATER | QS | QS | QS |
| DISODIUM EDTA | 0.1 | 0.1 | 0.1 |
| GLYCERINE | 10.0 | 5.0 | 15.0 |
| EMULGADE¹ | 0.1 | 0.2 | 0.2 |
| ISOHEXADECANE | 3.0 | 3.0 | 3.0 |
| ETHYL PARABEN | 0.15 | 0.15 | 0.15 |
| PROPYL PARABEN | 0.07 | 0.07 | 0.07 |
| STEARIC ACID | 0.1 | 0.1 | 0.1 |
| PEG-100 STEARATE | 0.1 | 0.1 | 0.1 |
| STEARYL ALCOHOL | 0.50 | 0.641 | 0.641 |
| CETYL ALCOHOL | 0.65 | 0.515 | 0.515 |
| BEHYNYL ALCOHOL | 0.32 | 0.420 | 0.420 |
| ISOPROPYL ISOSTEARATE | 1.10 | 1.50 | 1.95 |
| COCONUT OIL FRACTIONATED | 0.3 | 0.4 | 0.5 |
| DL-α TOCOPHEROL ACETATE | 0.25 | 0.50 | 0.15 |
| PETROLATUM | 2.0 | 2.0 | 2.0 |
| MICROTHENE² | 0.5 | 0.75 | 0.25 |
| DRY FLO PLUS³ | 0.5 | 0.5 | 0.5 |
| Prestige MICA SILVER STAR | 0.18 | 0.18 | 0.18 |
| Prestige MICA BRIGHT SILVER⁴ | 0.18 | 0.18 | 0.18 |
| Prestige MICA BRIGHT GOLD⁴ | 0.24 | 0.24 | 0.24 |
| NIACINAMIDE (Vitamin B3 compound) | 3.5 | 4.0 | 1.5 |
| PANTHENOL | 0.5 | 0.5 | 0.5 |
| PRODEW 400⁵ | 0.1 | 0.1 | 0.1 |
| CAMOMILE EXTRACT | 0.1 | 0.1 | 0.1 |
| SODIUM HYDROXIDE | 0.011 | 0.011 | 0.011 |
| *LUVIGEL⁶ EM (+) total | 1.50 | 2.00 | 2.50 |
| BENZYL ALCOHOL | 0.25 | 0.25 | 0.25 |
| DC 1503⁷ | 0.8 | 1.0 | 1.5 |
| PERFUME | 0.3 | 0.3 | 0.3 |

| | | | |
|---|---|---|---|
| 1. Emulgade :Supplied by Cognis Deutchland GmbH, Paul-Thomas Strasse 56, D-40551 Dusseldorf, Germany. | | | |
| 2. Microthene: Supplied by Equistar Chemicals, 1221 McKinney Street, Suite 700, Houston, TX 77252-2583 | | | |
| 3. Dry Flo : Supplied by National Starch Chemical Company, 10, Finderne Avenue, Bridgewater, NJ 08807, USA | | | |
| 4. Mica pearls: Supplied by Eckart Gmbh and Co., Kaiserstrasse 30, 90763 Fuerth, Germany. | | | |
| 5. Prodew 400: Supplied by Ajinomoto, Stubbenhuk 3, D-20459, Hamburg, Germany. | | | |
| 6. Luvigel⁸ EM - 97% + Volpo⁹ L3 Special 2.25% + Laureth 7 - 0.25% (Luvigel EM: Supplied by BASF P1c, PO Box 4-Earl Road, Cheadle Hulme, Cheshire SK8 6QG; Volpo 3 : Supplied by Croda Oleochemicals, Cowick hall, Snaith Goole, East Yorkshire, Dn14 9AA | | | |
| 7. DC 1503: Supplied by Dow Coming, Kings Court, 185 Kinds Rd, Reading, Berks, RGI 4EX | | | |

### Performance test

In clinical testing, 3-4 weeks usage of a composition with the same amount of Vitamin B3 compound and moisturizers as in Example 1 was shown to provide a very significant potentiation of SC barrier function, notably in response to chemical (SLS) and mechanical (tape-stripping) insult, superior relative to both no-treatment control, but also to products chosen to represent the labeled highest efficacy variants of the leading mass market brands of moisturising body lotions available in US and European geographies. As regards SC hydration and visual dry skin grading, excellent efficacy was also noted, particularly within the critical first 2-3 weeks of application (again, superior to other high-efficacy products). Lastly, the composition was also shown to significantly increase SC exfoliation rate relative to both no-treatment control and other products tested, using an improved measurement of dansyl chloride stain washout.

The clinical data clearly demonstrated that the composition was indeed addressing the pivotal endpoint of SC barrier function, with concurrent hydration and visual dry skin benefits. The composition provided significant attenuation of the dry skin cycle, producing body skin that is better able to cope with the rapidly changing environments in our modern world.

## Claims

1. Use of a skin care cosmetic composition comprising:
i) a moisturizer, preferably glycerine, and
ii) a vitamin B3 compound, preferably Niacinamide,
for breaking the cycle of dry skin.

2. Use according to claim 1 wherein the composition further comprises D-panthenol.

3. Use according to claim 1 or 2 wherein the composition further comprises a least one Natural Moisturizing Factor (NMF) selected from glycine, alanine, proline, serine, threonine, arginine, lysine, glutamic acid, sodium-2-pyrrolidone-5-carboxylate, and mixtures thereof.

4. A packaging containing a composition, said composition comprising
i) a moisturizer, preferably glycerine, and
ii) a vitamin B3 compound, preferably Niacinamide,
for breaking the cycle of dry skin,
wherein the packaging displays an indication to the user that the composition contained in the package breaks the cycle of dry skin.

5. A container according to claim 4 wherein the composition further comprises D-panthenol.

6. A container according to claim 4 or 5 wherein the composition further comprises a least one Natural Moisturizing Factor (NMF) selected from glycine, alanine, proline, serine, threonine, arginine, lysine, glutamic acid, sodium-2-pyrrolidone-5-carboxylate and mixtures thereof.

7. Use of a composition comprising:
i) a moisturizer, preferably glycerine, and
ii) a vitamin B3 compound, preferably Niacinamide,
for manufacture of a therapeutic composition for breaking the cycle of dry skin.

8. Use according to claim 7 wherein the composition further comprises D-panthenol.

9. Use according to claim 7 or 8 wherein the composition further comprises a least one Natural Moisturizing Factors (NMF) selected from glycine, alanine, proline, serine, threonine, arginine, lysine, glutamic acid, sodium-2-pyrrolidone-5-carboxylate and mixtures thereof.
